# EUROPÄISCHE PATENTSCHRIFT

(11) **EP 0 731 671 B1**
(45) Veröffentlichungstag und Bekanntmachung des Hinweises auf die Patenterteilung: **01.04.1998**
(21) Anmeldenummer: 95901438.2
(22) Anmeldetag: 26.11.1994
(51) Int. Cl.: A61B 17/12

(54) **CHIRURGISCHES INSTRUMENT ZUM ABKLEMMEN EINES GEFÄSSES ODER DERGLEICHEN**
SURGICAL INSTRUMENT USED TO CLAMP A VESSEL OR THE LIKE
INSTRUMENT CHIRURGICAL SERVANT A CLAMPER UN VAISSEAU OU SIMILAIRE

(30) Priorität: 01.12.1993 DE 4340821
(43) Veröffentlichungstag der Anmeldung: 18.09.1996
(73) Patentinhaber: KLINIKUM DER ALBERT-LUDWIGS-UNIVERSITÄT FREIBURG, D-79106 Freiburg (DE)
(72) Erfinder: MATERN, Ulrich, D-79283 Bollschweil (DE)
(74) Vertreter: Schmitt, Hans, Dipl.-Ing.
(86) Internationale Anmeldenummer: EP9403916
(87) Internationale Veröffentlichungsnummer: WO9515121

(56) Entgegenhaltungen:
- WO-A-90/06725
- DE-A- 3 504 202
- DE-C- 4 228 910
- US-A- 5 207 694

## Beschreibung

Die Erfindung bezieht sich auf ein chirurgisches Instrument zum Abklemmen (Ligatur) eines Gefäßes, Hohlorganes oder dergleichen Körperteil mittels eines Fadens oder Zügels, insbesondere bei der minimalinvasiven Chirurgie.

Bei der Ligatur, also der Unterbindung von Gefäßen und dergleichen wird zum Umschlingen des Gefäßes als sogenannter Zügel ein Faden, ein Kunststoffschlauch oder ein Gummifaden verwendet. Dieser Zügel wird um das abzubindende Gefäß geschlungen und dann verknotet.
Weiterhin besteht die Möglichkeit, zum Unterbinden eine Klemmzange zu verwenden. Diese kann aber nur kurzzeitig verwendet werden, da diese im Operationsfeld hinderlich ist. Während bei eröffnetem Abdomen eine Ligatur vergleichsweise einfach durchführbar ist, ergeben sich bei geschlossenem Abdomen und über Trokarhülsen eingeführten Instrumenten erhebliche Probleme, da der Bewegungsspielraum stark eingeschränkt ist.
Daraus resultiert ein hoher Zeitaufwand für das Anlegen der Ligatur. Nachteilig ist auch, daß der Ligaturzug nach dem Verknoten nicht mehr verändert werden kann.
Aus der WO 90/06725 ist ein Ligatursystem bekannt, das in Verbindung mit Trokaren einsetzbar ist und das einen Faden aufweist, der mit seinem Ende in eine Höhlung einziehbar und dort durch Haltemittel, insbesondere Rastprofilierungen festlegbar ist. Zum Einziehen des Fadenendes ist ein durch eine Trokarhülse zuführbares Zugelement vorgesehen, mit dem das Fadenende erfaßt und die Schlinge zugezogen werden kann.
Auch hierbei ist ein Lockern der Ligatur nicht möglich.
Beim Abbinden eines Blutgefäßes oder dergleichen kann deshalb nicht durch Lockern der Ligatur überprüft werden, ob zum Beispiel eine Gefäßnaht dicht ist. Auch ein zeitweises Durchbluten oder ein zeitweises Perfundieren eines Organes ist somit auf einfache Weise nicht möglich. Es muß deshalb für solche Fälle in aufwendiger Weise die erste Ligatur durchtrennt und dann bedarfsweise wieder eine neue Ligatur angelegt werden.

Aufgabe der vorliegenden Erfindung ist es, ein chirurgisches Hilfsinstrument der eingangs erwähnten Art zu schaffen, mit dessen Hilfe das Anlegen einer Ligatur wesentlich vereinfacht und in kurzer Zeit vorgenommen werden kann. Dies soll insbesondere auch bei der minimalinvasiven Chirurgie und dem Operieren durch Trokarhülsen oder dergleichen hindurch möglich sein. Darüberhinaus soll die Ligatur auch vorübergehend gelockert und auch wieder angezogen werden können.

Zur Lösung dieser Aufgabe wird erfindungsgemäß insbesondere vorgeschlagen, daß das Instrument eine Plombe mit einem Fadenhalter sowie einen mit der Plombe kuppelbaren, schaftartigen Plombenhalter aufweist und daß der Plombenhalter Betätigungselemente zum Öffnen beziehungsweise Schließen des Plomben-Fadenhalters aufweist.
Mit diesem Instrument ist auf einfache Weise eine Ligatur durchführbar, indem die Plombe mit Hilfe des Plombenhalters an das zu unterbindende Gefäß gebracht und dort der das Gefäß umschlingende Faden von der Plombe klemmend gehalten wird. Der Plombenhalter kann dann entfernt werden, so daß er im Operationsfeld nicht hinderlich im Weg ist. Andererseits kann der Plombenhalter auch wieder mit der Plombe zum Entfernen oder Lockern der Ligatur gekoppelt werden. Das Anlegen der Ligatur und auch ein eventuelles Entfernen kann mit diesem Instrument auch besonders schnell durchgeführt werden, was bei einer Reihe von Operationen von besonderer Bedeutung ist. Vorteilhaft ist weiterhin, daß die Handhabung auch unter sehr beengten Verhältnissen problemlos möglich ist.

Zweckmäßigerweise bilden die Betätigungselemente des Plombenhalters gleichzeitig Kupplungselemente zum Verbinden mit der Plombe. Dies trägt mit zu einer einfachen Handhabung des Instrumentes bei. Ist der Plombenhalter mit der Plombe gekuppelt, so wird gleichzeitig der Fadenhalter in Offenstellung gehalten. Beim Abkuppeln geht der Fadenhalter automatisch in Klemmstellung und hält dann das dazwischen befindliche Zügelende fest. Eine zusätzliche Betätigung zum Klemmen beziehungsweise Freigeben des Zügelendes ist bei dieser Ausführungsform nicht erforderlich.

Nach einer Weiterbildung der Erfindung ist vorgesehen, daß der Fadenhalter der Plombe Klemmelemente zum Halten wenigstens eines Zügel- oder Fadenschlingenendes aufweist und daß vorzugsweise ein Fadenende an der Plombe fixiert und das andere Fadenende von den Klemmelementen gehalten ist. Somit wird das freie Fadenende zunächst um das abzuklemmende Gefäß oder dergleichen geführt und dann von den Klemmelementen nach dem Trennen von Plombe und Plombenhalter festgehalten.

Besonders vorteilhaft ist es, wenn die Klemmelemente entgegen der Spannrichtung des Fadens selbstsperrend ausgebildet beziehungsweise angeordnet sind und daß die Klemmelemente in Klemmrichtung federbeaufschlagte Klemmbacken aufweisen, die zueinander schwenkbar gelagert und durch die Fadenspannung in Klemmrichtung beaufschlagbar sind.
Durch diese Ausbildung der Klemmeinrichtung beziehungsweise des Fadenhalters kann der Faden auch nach dem Abkuppeln des Plombenhalters noch nachgezogen und gestrafft werden und so die Fadenspannung besonders gut den jeweiligen Bedürfnissen angepaßt werden.

Vorzugsweise weist die Plombe einen insbesondere zentralen Durchgangskanal für ein Fadenende auf. Durch diesen Durchgangskanal kann das lose Fadenende zur Bildung einer Schlinge hindurchgezogen werden.
Zweckmäßigerweise weist dabei der Plombenhalter, insbesondere dessen Schaft einen vorzugsweise zentralen Durchgangskanal auf, der in Kuppelstellung von Plombe und Plombenhalter mit dem der Plombe fluchtet und daß eine durch diese Durchgangskanäle passende, vom äußeren Ende des Plombenhalters einführbare Faßzange vorgesehen ist.
Mit der durch den Plombenhalter und die Plombe hindurchgeführten Faßzange oder dergleichen kann das freie, um das abzuklemmende Gefäß geschlungene Fadenende erfaßt und durch die Klemmvorrichtung hindurchgeführt werden. Nach dem Abkuppeln des Plombenhalters kann dann durch Zug am Fadenende die Schlingenweite passend eingestellt werden.

Eine Ausführungsform der Erfindung sieht vor, daß der Plombenhalter als Betätigungs- und Halteelemente zangenartige Faßbacken aufweist, die vorzugsweise mit ihren Innenseiten etwa an die Außenkontur der Plombe angepaßt sind und in Schließstellung die Betätigungsvorsprünge zum Lösen der Fadenklemmung beaufschlagen. Der Plombenhalter weist dadurch einen vergleichsweisen geringen Außenquerschnitt auf und läßt sich dadurch gut zum Beispiel auch durch Trokarhülsen einführen.

Eine andere Ausführungsform der Erfindung sieht vor, daß die Klemmelemente des Fadenhalters als Spreizbacken ausgebildet sind und zwischen sich einen Durchgangskanal für den Faden aufweisen, daß außenseitig an den Spreizbacken ein mit einer verschiebbaren Klemmhülse zusammenwirkender Klemmkonus vorgesehen ist, daß die Klemmhülse in Schließrichtung federbeaufschlagt ist und daß an den Klemmelementen sowie dem Klemmkonus Angriffsstellen für den Plombenhalter zum Öffnen und Schließen der Klemmelemente vorgesehen ist.
Die Klemmung des Fadens erfolgt hier durch Beaufschlagung der Klemmelemente von außen mit Hilfe der verschiebbaren, federbeaufschlagten Klemmhülse.

Zusätzliche Ausgestaltungen der Erfindung sind in den weiteren Unteransprüchen aufgeführt. Nachstehend ist die Erfindung mit ihren wesentlichen Einzelheiten anhand der Zeichnungen noch näher erläutert.

Erfindung mit ihren wesentlichen Einzelheiten anhand der Zeichnungen noch näher erläutert.

Es zeigt:
- Fig. 1: eine Seitenansicht eines erfindungsgemäßen Instrumentes mit Plombe und Plombenhalter in getrennter Lage,
- Fig. 2: die in Figur 1 gezeigte Plombe in vergrößerter Darstellung,
- Fig. 3: eine Stirnseitenansicht der in Figur 2 gezeigten Plombe bei geschlossenem Fadenhalter,
- Fig. 4: eine etwa Figur 3 entsprechende Stirnseitenansicht der Plombe, hier jedoch mit geöffnetem Fadenhalter,
- Fig. 5: eine Seitenansicht des inneren Endes des Plombenhalters und
- Fig. 6: eine etwa Figur 5 entsprechende Darstellung, hier jedoch mit durch den Plombenhalter hindurchgeführter Faßzange.

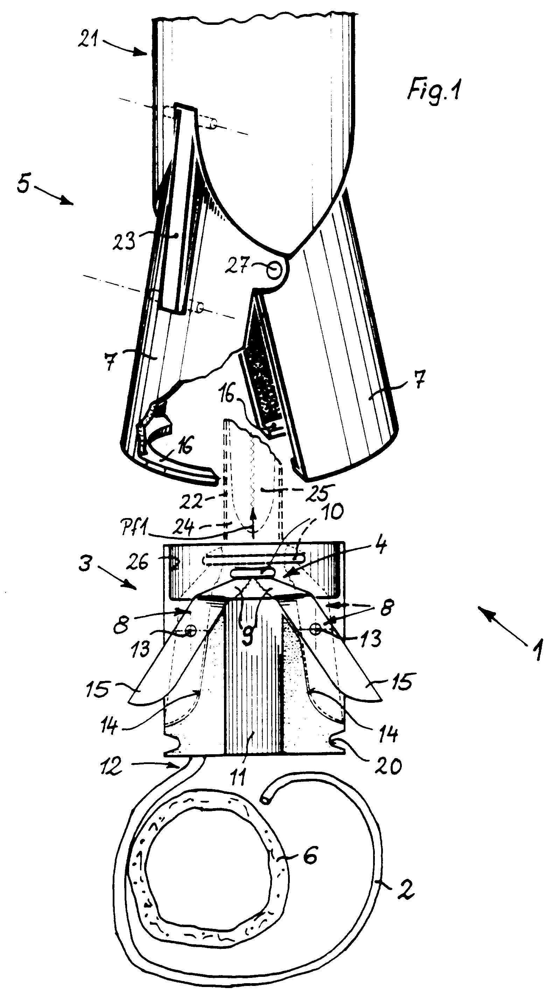

Ein chirurgisches Instrument 1 (Figur 1) dient zum Abklemmen eines Gefäßes oder dergleichen mittels eines Fadens 2 (Zügel) und weist im wesentlichen eine Plombe 3 mit einem Fadenhalter 4 sowie einem hier nur teilweise dargestellten Plombenhalter 5 auf.
Zum Positionieren der Plombe 3 bei einem abzuklemmenden Gefäß 6 ist die Plombe 3 mit dem Plombenhalter 5 kuppelbar. Der Plombenhalter 5 weist dazu Faßbacken 7 auf, die greiferartig öffenbar und schließbar sind. In Figur 1 sind die Faßbacken 7 in Offenstellung beziehungsweise Greifstellung dargestellt, so daß der Plombenhalter 5 mit diesen Faßbacken über die Plombe 3 geschoben werden kann.

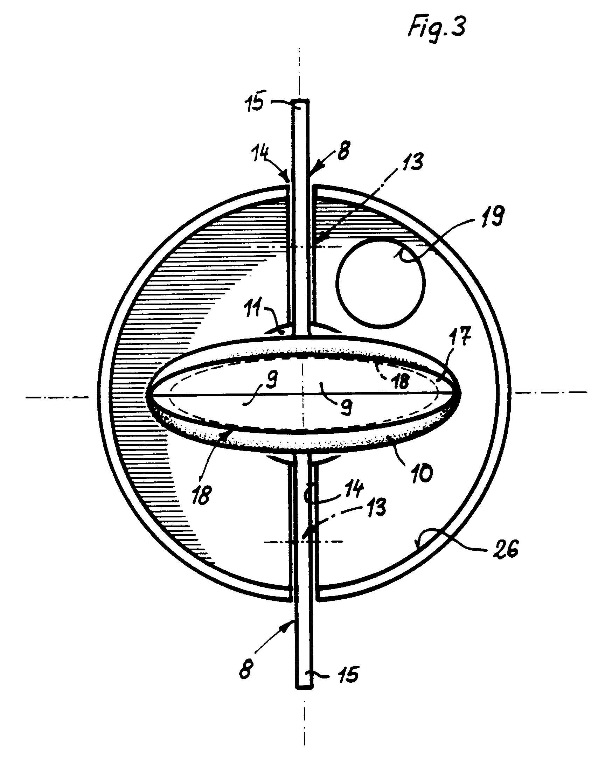

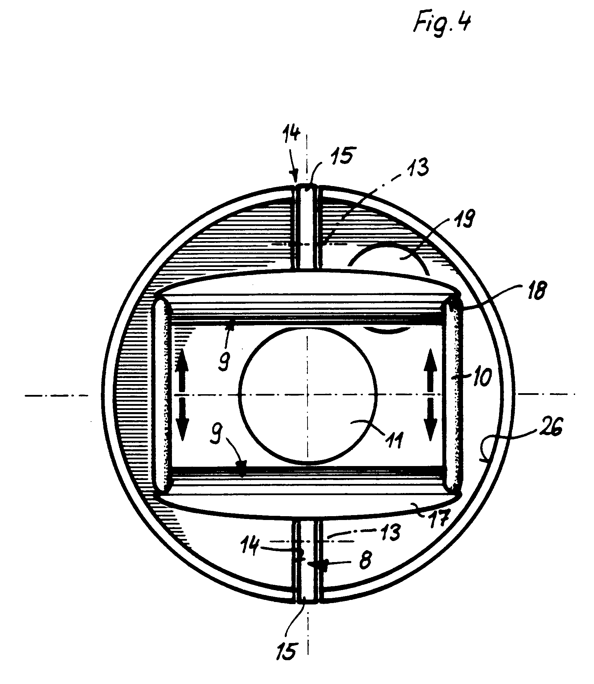

Die Faßbacken 7 bilden gleichzeitig Betätigungselemente zum Öffnen beziehungsweise Schließen des Plomben-Fadenhalters 4. Im gezeigten Ausführungsbeispiel ist der Fadenhalter 4 prinzipiell wie eine sogenannte Curry-Klemme ausgebildet. Dazu sind Klemmelemente 8 vorgesehen, die als Schwenkhebel ausgebildet sind und an ihren inneren Enden federbeaufschlagte Klemmbacken 9 aufweisen. Mit diesen Klemmbacken 9 wird ein dazwischen befindlicher Faden 2 festgeklemmt. Durch die Lage und Ausbildung der Klemmelemente 8 wird der durchgeführte Faden 2 selbstsperrend gehalten, so daß er einerseits in Spannrichtung gemäß dem Pfeil Pf 1 durch die Klemmelemente 8 hindurchgezogen werden kann, andererseits aber entgegen dieser Spannrichtung ein sich selbstverstärkendes Klemmen des Fadens und damit ein sicheres Halten erfolgt. Die Klemmelemente 8 sind durch einen Gummiring 10 an ihren Klemmbacken-Enden beaufschlagt, so daß sich in entlasteter Lage die in Figur 1 gezeigte, durchgezogene Stellung der Klemmelemente 8 einstellt.
Die Plombe 3 weist einen zentralen Durchgangskanal 11 auf, durch den das eine Fadenende hindurchgeführt werden kann und dann auch zwischen den in diesen Durchgangskanal 11 eingreifenden Faßbacken 7 verläuft (vgl. auch Figur 3 und 4). Das andere Fadenende 12 ist an der Plombe 3 fixiert, so daß bei einem durch die Plombe beziehungsweise deren Durchgangskanal 11 hindurchgezogenem, freien Fadenende eine Schlinge gebildet ist.

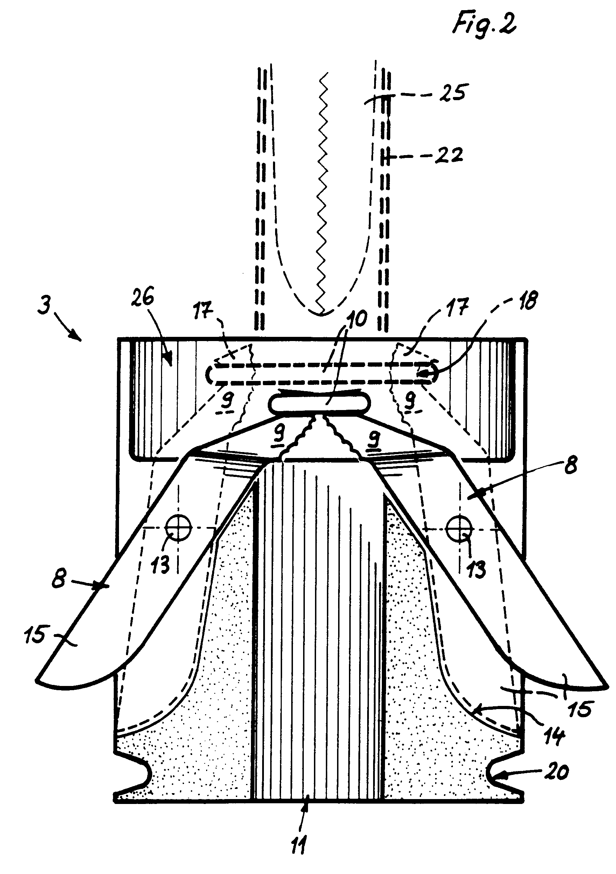

Die schwenkhebelartigen Klemmelemente 8 weisen Schwenklagerungen 13 in der Wand des Plombenkörpers auf, wobei diese Schwenklager 13 schlitzförmige Taschen 14 für die Klemmelemente 8 in dem Plombengehäuse durchgreifen.
Die Klemmelemente weisen an ihren den Klemmbacken 9 abgewandten Enden sich über die Schwenklager 13 fortsetzende Betätigungsvorsprünge 15 auf. Diese stehen in der in Figur 1 und 2 durchgezogen dargestellten Lage der Klemmelemente 8 über den Außenumfang der Plombe 3 vor (vgl. auch Figur 3).

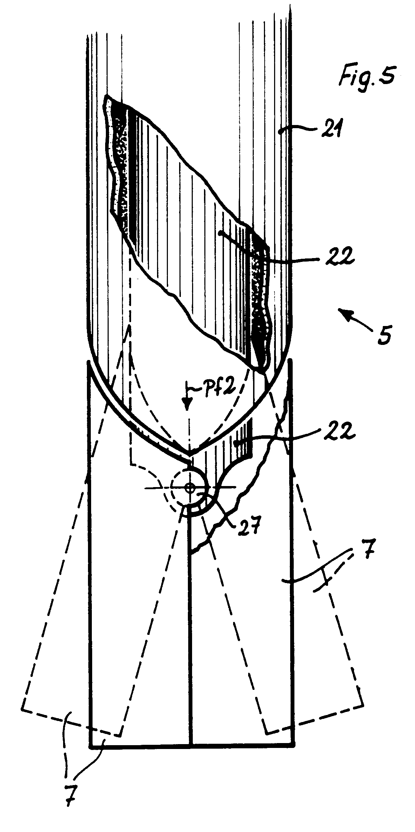

Wird nun der Plombenhalter mit seinen Faßbacken 7 über die Plombe 3 geschoben und dann die Faßbacken in die in Figur 5 gezeigte Schließstellung gebracht, werden die Betätigungsvorsprünge 15 der Klemmelemente 8 beaufschlagt und nach innen in die Taschen 14 eingeschwenkt. Gleichzeitig wird der Fadenhalter 4 gegen die Rückstellkraft des Gummiringes 10 geöffnet. Diese Stellung der Klemmelemente 8 ist in Figur 1 und 2 strichliniert eingezeichnet.
Die Faßbacken 7 haben an ihren freien Enden nach innen weisende Abkröpfungen 16, die in Schließstellung der Faßbacken 7 in eine Ringnut 20 der Plombe 3 eingreifen. Die Plombe 3 und der Plombenhalter 5 sind in dieser Lage miteinander gekuppelt.

Im Bereich der Klemmbacken 9 weist der Durchgangskanal 11 eine Erweiterung 26 auf, so daß genügend Platz für die Aufspreizung der Klemmbacken 9 und auch für das Rückstellelement (Gummiring 10) vorhanden ist. Wie gut in den Figuren 3 und 4 erkennbar, schließen sich an die Klemmbacken 9 am lagerfernen Ende laschenförmige Anformungen 17 an, die Nutabschnitte 18 für die Aufnahme des Gummiringes 10 bilden.
In den Figuren 3 und 4 ist neben dem Durchgangskanal 11 noch ein weiterer Kanal 19 erkennbar, durch den das zu befestigende Fadenende 12 geführt und dort gegebenenfalls auch fixiert werden kann. Im einfachsten Falle wird das Fadenende 12 von unten durch die Plombe 3 beziehungsweise den Kanal 19 hindurchgesteckt und an der anderen Seite verknotet.

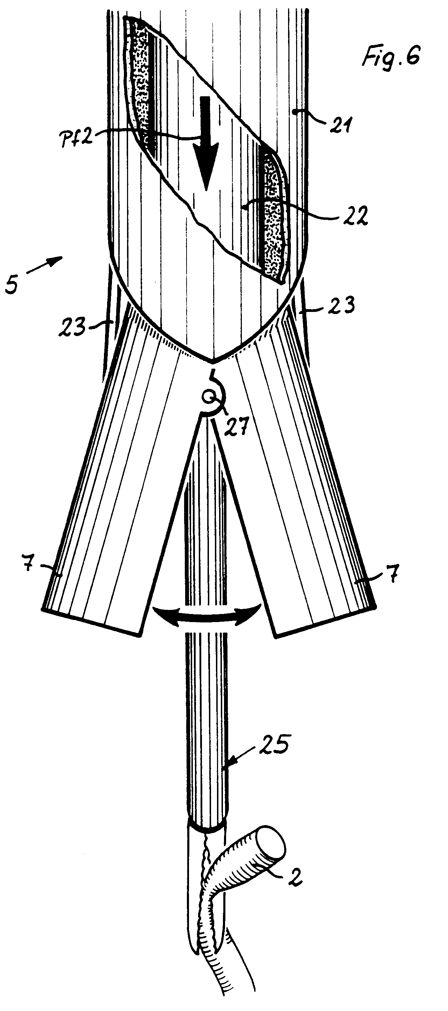

Der Plombenhalter 5 ist schaftartig ausgebildet und weist eine an die jeweiligen Operationsverhältnisse angepaßte Länge auf. Am inneren Ende befinden sich die als Faßbacken 7 ausgebildeten Betätigungselemente zur Beaufschlagung der Betätigungsvorsprünge 15 der Klemmelemente 8, wobei diese Betätigungselemente gleichzeitig auch Kupplungselemente zum Verbinden mit der Plombe 3 bilden. Für das Verbinden greifen die Abkröpfungen 16 der Faßbacken 7 in die Ringnut 20 der Plombe 3 ein. Am äußeren Ende des Plombenhalters 5 können hier nicht dargestellte, griffartige Betätigungsmittel zum Öffnen und Schließen der Faßbacken 7 vorgesehen sein. Die Übertragungsmittel für diese Bewegung verlaufen im Inneren des Plombenhalters 5. Dieser weist ein rohrförmiges Schaftteil 21 auf. In diesem rohrförmigen Schaftteil 21 ist ein Innenrohr 22 geführt, das in Längsrichtung verschiebbar ist. Diese Verschiebebewegung erfolgt vom äußeren Ende her zum Beispiel mittels eines entsprechenden Griffteiles.
Wie insbesondere in Figur 5 erkennbar, sind die Schwenklager 27 der Faßbacken 7 am inneren Ende dieses Innenrohres 20 vorgesehen. Dieses Ende des Innenrohres 22 mit den Schwenklagern 27 steht etwas über das innere Ende des ein Außenrohr bildenden Schaftteiles 21 vor.
Die Faßbacken 7 bilden in Schließstellung (Figur 5) von ihrer Außenkontur her eine Verlängerung des Schaftteiles 21 und sind jeweils durch etwa in Längsrichtung halbierte, rinnenförmige Rohrabschnitte gebildet. Die Schwenklager 27 befinden sich nahe dem Ende des Schaftteiles 21 im Bereich der Mittellängsebene, so daß die Faßbacken 7 greiferartig in Greifstellung aufschwenkbar sind, wie dies in Figur 6 und strichliniert in Figur 5 gezeigt ist.
Damit diese Greiferbewegung synchron von beiden Faßbacken 7 bei einer Längsverschiebung des Innenrohres 22 erfolgt, sind jeweils die Faßbacken 7 mit Seitenabstand zur Schwenklagerachse mit Zug- und Druckstegen 23 (vgl. Figur 1) gekuppelt, die ihrerseits mit ihren anderen Enden mit dem Schaftteil 21 verbunden sind. Dadurch ist eine Zwangssteuerung gebildet, durch die bei einer Relativverschiebung von Innenrohr 22 zu Schaftteil 21 ein Auf- und Zuschwenken der Faßbacken 7 bewirkt wird.
Zum Aufschwenken der Faßbacken in Greifstellung (Figur 6) wird das Innenrohr 22 etwas in Richtung des Pfeiles Pf 2, also zum inneren Ende hin verschoben, so daß auch die Schwenklagerachse weiter nach außen verlagert wird. Da die Faßbacken 7 außenseitig im vorliegenden Falle über gelenkig einerseits mit den Faßbacken 7 und andererseits mit dem Schaftteil 21 angelenkten Zug- und Druckstegen 23 verbunden ist (Figur 1), erfolgt gleichzeitig mit der Verlagerung der Schwenklager 27 nach außen ein Aufschwenken der Faßbacken 7 in Greifstellung. Anstatt der vorgesehenen Zug- und Druckstege 23 könnten hier auch für die Aufschwenkbewegung wirksame Zugelemente vorgesehen sein und für die Schließbewegung können zwischen dem Schaftteil und den Faßbacken wirksame Anschläge vorgesehen sein.

Für das Anlegen einer Ligatur wird folgendermaßen vorgegangen:
In Ausgangsstellung ist die Plombe 3 mit dem Plombenhalter 5 verbunden und von den Faßbacken 7 gehalten. Der Faden 2 ist mit seinem einen Ende 12 mit der Plombe 3 verbunden. Diese Instrumentenanordnung wird dann mit dem inneren Plombenende voran in den Bereich des zu unterbindenden Gefäßes 6 gebracht.
Das Innenrohr 22 des Plombenhalters 5 weist eine längsdurchgehende Innenhöhlung 24 auf, was in Figur 1 und 2 strichliniert angedeutet ist. Durch diese Innenhöhlung 24 und die damit in Kuppelstellung von Plombenhalter und Plombe fluchtenden Durchgangskanal 11 der Plombe kann nun eine Faßzange 25 (Figur 1, 2 und 6) hindurch geführt werden und damit das freie Ende des um das Gefäß 6 geschlungenen Fadens 2 erfaßt werden. Da in dieser Lage die Betätigungsvorsprünge 15 der Klemmelemente 8 von den Faßbacken 7 beaufschlagt sind, befinden sich auch die Klemmbacken 9 in Offenstellung, wie dies strichliniert in Figur 1 und 2 und auch in Figur 4 gezeigt ist. Die Faßzange 25 kann somit in dieser Lage ungehindert durch das Instrument hindurchgeführt werden.
Das von der hindurchgeführten Faßzange 25 ergriffene, freie Fadenende wird anschließend durch die Plombe 3 hindurchgezogen. Der Plombenhalter 5 kann dann durch Öffnen der Faßbacken 7 von der Plombe 3 getrennt werden, wobei die Klemmelemente 8 automatisch in Klemmstellung schwenken und der durchgezogene Faden zwischen den Klemmbacken 9 festgeklemmt wird. Mit der Faßzange 25 kann der Faden 2 in Richtung des Pfeiles Pf 1 gestrafft und der Fadenzug den jeweiligen Verhältnissen angepaßt werden.

Andererseits ist durch die spezielle Konstruktion des Fadenhalters 4 (Curryklemme) verhindert, daß sich die Fadenspannung lockert, da die Klemmelemente 8 selbstsperrend wirken. Ist die passende Faden- oder Zügelspannung eingestellt, verbleibt nur noch die Plombe 3 im Bereich des abgeklemmten Gefäßes 6.
Die Zügelspannung kann bedarfsweise auch wieder gelockert werden. Dazu wird das durch die Plombe 3 hindurchgezogene Fadenende mit der durch den Plombenhalter 5 geführten Faßzange 25 ergriffen, so wie dies in Figur 6 gezeigt ist. Der etwas gestraffte Faden kann dann als " Leitschnur " für den Plombenhalter 5 dienen, der entlang der Faßzange und dem davon gehaltenen Faden problemlos und schnell auf die Plombe 3 geschoben wird.
Mit Hilfe des Plombenhalters 5 werden die Klemmelemente 8 verschwenkt, so daß der Faden 2 freigegeben wird und soweit gelockert werden kann, daß beispielsweise eine Durchblutung des Gefäßes 6 möglich ist. Bedarfsweise kann anschließend der Zügel zum Abklemmen des Gefäßes wieder angezogen werden.

Wie bereits vorerwähnt, bilden die Faßbacken 7 in Schließstellung (Figur 5) eine Verlängerung des Schaftteiles 21, wobei auch im Bereich dieser Faßbacken 7 keine Querschnittserweiterung vorhanden ist. Auch bei aufgenommener und gekuppelter Plombe 3 bildet das Instrument 1 insgesamt ein im wesentlichen glattes, schlankes Rohrstück, so daß es besonders gut auch bei Operationen durch Trokarhülsen hindurch einsetzbar ist.

## Patentansprüche

1. Chirurgisches Instrument zum Abklemmen eines Gefäßes, Hohlorganes oder dergleichen Körperteil mittels eines Fadens oder Zügels, insbesondere bei der minimalinvasiven Chirurgie, **dadurch gekennzeichnet,** daß das Instrument (1) eine Plombe (3) mit einem Fadenhalter (4) sowie einen mit der Plombe kuppelbaren schaftartigen Plombenhalter (5) aufweist und daß der Plombenhalter Betätigungselemente (7) zum Öffnen beziehungsweise Schließen des Plomben-Fadenhalters aufweist.

2. Instrument nach Anspruch 1, dadurch gekennzeichnet, daß die Betätigungselemente des Plombenhalters (5) gleichzeitig Kupplungselemente zum Verbinden mit der Plombe (3) bilden.

3. Instrument nach Anspruch 1 oder 2, dadurch gekennzeichnet, daß der Fadenhalter (4) der Plombe (3) Klemmelemente (8) zum Halten wenigstens eines Zügel- oder Fadenschlingenendes aufweist und daß vorzugsweise ein Fadenende (12) an der Plombe (3) fixiert und das andere Fadenende von den Klemmelementen (8) gehalten ist.

4. Instrument nach Anspruch 3, dadurch gekennzeichnet, daß die Klemmelemente (8) entgegen der Spannrichtung des Fadens (2) selbstsperrend ausgebildet beziehungsweise angeordnet sind und daß die Klemmelemente in Klemmrichtung federbeaufschlagte Klemmbacken (9) aufweisen, die zueinander schwenkbar gelagert und durch die Fadenspannung in Klemmrichtung beaufschlagbar sind.

5. Instrument nach Anspruch 3 oder 4, dadurch gekennzeichnet, daß die Klemmelemente (8) von den Betätigungselementen des Plombenhalters (5) beaufschlagbare Betätigungsvorsprünge (15) zum Lösen der Fadenklemmung aufweisen.

6. Instrument nach einem der Ansprüche 1 bis 5, dadurch gekennzeichnet, daß die Plombe (3) einen vorzugsweise zentralen Durchgangskanal (11) für ein Fadenende beziehungsweise ein Greifinstrument (25) aufweist.

7. Instrument nach einem der Ansprüche 1 bis 6, dadurch gekennzeichnet, daß der Plombenhalter als Betätigungs- und Halteelemente zangenartige Faßbacken (7) aufweist, die vorzugsweise mit ihren Innenseiten etwa an die Außenkontur der Plombe (3) angepaßt sind und in Schließstellung die Betätigungsvorsprünge (15) zum Lösen der Fadenklemmung beaufschlagen.

8. Instrument nach Anspruch 7, dadurch gekennzeichnet, daß die Faßbacken (7) des Plombenhalters (5) am inneren Ende eines Schaftteiles (21) schwenkbar gelagert sind und daß am äußeren Ende des Plombenhalters Betätigungsmittel zum Öffnen und Schließen der Faßbacken 7 vorgesehen sind.

9. Instrument nach einem der Ansprüche 1 bis 8, dadurch gekennzeichnet, daß der Plombenhalter (5) einen vorzugsweise zentralen Durchgangskanal (24) aufweist, der in Kuppelstellung von Plombe (3) und Plombenhalter (5) mit dem der Plombe fluchtet und daß eine durch diese Durchgangskanäle passende, vom äußeren Ende des Plombenhalters (5) einführbare Faßzange (25) vorgesehen ist.

10. Instrument nach Anspruch 8 oder 9, dadurch gekennzeichnet, daß der Plombenhalter (5) als Übertragungs- und Betätigungsmittel zum Öffnen und Schließen der Faßbacken (7) vorzugsweise ein koaxial im Schaft geführtes Innenrohr (22) aufweist, an dessen inneren Ende Schwenklager (27) für die Faßbacken (7) vorgesehen sind und daß insbesondere an den Faßbacken zur Schwenklagerachse parallel geführte, mit der Außenhülse beziehungsweise dem äußeren Schaftteil (21) verschwenkbar verbundene Zug- und Druckstege (23) angreifen.

11. Instrument nach einem der Ansprüche 1 bis 10, dadurch gekennzeichnet, daß die Plombe (3) etwa zylinderförmig mit einem axialen, vorzugsweise zentralen Durchgangskanal (11) ausgebildet ist und daß die Klemmelemente (8) schwenkbar gelagert sind und mit ihren Klemmbacken (9) im Bereich des Durchgangskanales (11) angeordnet sind und mit ihren anderen, die Betätigungsvorsprünge (15) aufweisenden Enden vorzugsweise über den Außenumriß der Plombe (3) überstehen.

12. Instrument nach einem der Ansprüche 1 bis 11, dadurch gekennzeichnet, daß die Klemmelemente (8) durch wenigstens einen, vorzugsweise in Nutabschnitten (18) von Anformungen (17) gelagerten Gummiring (10) oder dergleichen in Schließstellung federbeaufschlagt sind.

13. Instrument nach einem der Ansprüche 1 bis 12, dadurch gekennzeichnet, daß die Plombe (3) einen vorzugsweise parallel zum zentralen Durchganskanal (11) verlaufenden, weiteren Kanal (19) für das andere Fadenende (12) aufweist.

14. Instrument insbesondere nach Anspruch 1, dadurch gekennzeichnet, daß die Klemmelemente (8) des Fadenhalters (4) als Spreizbacken ausgebildet sind und zwischen sich einen Durchgangskanal für den Faden aufweisen, daß außenseitig an den Spreizbacken ein mit einer verschiebbaren Klemmhülse zusammenwirkender Klemmkonus vorgesehen ist, daß die Klemmhülse in Schließrichtung federbeaufschlagt ist und daß an den Klemmelementen sowie dem Klemmkonus Angriffsstellen für den Plombenhalter zum Öffnen und Schließen der Klemmelemente vorgesehen sind.

15. Instrument nach Anspruch 14, dadurch gekennzeichnet, daß der Fadenhalter eine im Bereich der Spreizbacken längsgeschlitzte und dort im Außenquerschnitt vorzugsweise konisch erweiterte Hülse aufweist, auf der die ringförmige Klemmhülse verschiebbar gelagert ist.

16. Instrument nach Anspruch 14 oder 15, dadurch gekennzeichnet, daß eine der Spreizbacken sowie die Klemmhülse als Angriffsstellen für den Plombenhalter sich etwa in axialer Richtung erstreckende Verlängerungen aufweisen, an deren Enden sich vorzugsweise mit Durchtrittslöchern versehene, in Klemmstellung zueinander beabstandete Betätigungsflansche befinden.

## Claims

1. A surgical instrument used to clamp a vessel, hollow organ or similar part of the body by means of a thread or rein, particularly in minimum invasive surgery, **characterized in that** the instrument (1) has a plumb (3) with a thread holder (4) and a shaft-like plumb holder (5) that can be coupled with the plumb, and that the plumb holder has actuating members (7) for opening and closing the plumb thread holder.

2. An instrument as claimed in claim 1, characterized in that the actuating members of the plumb holder (5) at the same time compose coupling elements for connecting to the plumb (3).

3. An instrument as claimed in claim 1 or claim 2, characterized in that the thread holder (4) of the plumb (3) has clamping elements (8) for holding at least one loop end of the rein or thread, and that preferably one thread end (12) is fixed at the plumb (3) and the other thread end is held by the clamping elements (8).

4. An instrument as claimed in claim 3, characterized in that the clamping elements (8) are configured and arranged to be self-locking counter to the tensioning direction of the thread (2) and that the clamping elements have clamping jaws (9) spring-loaded in the clamping direction which are mounted for swivelling towards each other and can be acted upon in the clamping direction by the tension of the thread.

5. An instrument as claimed in claim 3 or claim 4, characterized in that the clamping elements (8) have actuating projections (15) that can be acted upon by the actuating elements of the plumb holder (5) to unclamp the thread.

6. An instrument as claimed in any one of claims 1 to 5, characterized in that the plumb (3) has a preferably central passage (11) for one thread end and for a gripping instrument (25).

7. An instrument as claimed in any one of claims 1 to 6, characterized in that the plumb holder has actuating and holding elements in the form of forcipated clasping jaws (7) preferably having their inner surfaces adapted generally to the outer contour of the plumb (3) and in the closed position engaging the actuating projections (15) to unclamp the thread.

8. An instrument as claimed in claim 7, characterized in that the clasping jaws (7) of the plumb holder (5) are swivel-mounted at the inner end of a shaft member (21) and that actuating means for opening and closing the clasping jaws (7) are provided at the outer end of the plumb holder.

9. An instrument as claimed in any one of claims 1 to 8, characterized in that the plumb holder (5) has a preferably central passage (24) in alignment with that of the plumb in the coupled position of plumb (3) and plumb holder (5), and that forceps (25) are provided which fit through said passages and can be inserted from the outer end of the plumb holder (5).

10. An instrument as claimed in claim 8 or claim 9, characterized in that the plumb holder (5) preferably has coaxially contained in the shaft an inner tube (22) as transferring and actuating means for opening and closing the clasping jaws (7), the inner end of said tube being provided with swivel bearings (27) for the clasping jaws (7), and that in particular pull-push arms (23) which are guided parallel to the axis of the swivel bearings and are swivel-connected to the outer sleeve and outer shaft member (21) act upon the clasping jaws.

11. An instrument as claimed in any one of claims 1 to 10, characterized in that the plumb (3) is of substantially cylindrical configuration with an axial, preferably central passage (11) and that the clamping elements (8) are swivel-mounted and are arranged with their clamping jaws (9) in the region of the passage (11) and with their other ends exhibiting the actuating projections (15) are preferably proud of the outer contour of the plumb (3).

12. An instrument as claimed in any one of claims 1 to 11, characterized in that the clamping elements (8) are spring-loaded into the closing position by at least one rubber ring (10) or the like preferably mounted in grooved portions (18) of tongues (17).

13. An instrument as claimed in any one of claims 1 to 12, characterized in that the plumb (3) has a further passage (19) preferably extending parallel to the central passage (11) and serving for the other thread end (12).

14. An instrument, particularly as claimed in claim 1, characterized in that the clamping elements (8) of the thread holder (4) take the form of expanding jaws and have a passage therebetween for the thread, that the expanding jaws are provided with an external clamping cone co-operating with a slidable clamping sleeve, that the clamping sleeve is spring-loaded in the closing direction and that the clamping elements and the clamping cone are provided with contact points for the plumb holder for opening and closing the clamping elements.

15. An instrument as claimed in claim 14, characterized in that the thread holder has a sleeve which is longitudinally slotted in the region of the expanding jaws and there preferably has a flared outside cross section, upon which sleeve the annular clamping sleeve is slidably mounted.

16. An instrument as claimed in claim 14 or 15, characterized in that one of the expanding jaws and the clamping sleeve have substantially axially extending extensions as contact points for the plumb holder, and that actuating flanges in spaced relationship to one another in the clamping position and preferably provided with openings therethrough are located at the ends of said extensions.

## Revendications

1. Instrument chirurgical pour clamper un vaisseau, un organe creux ou des parties du corps analogues au moyen d'un fil ou d'une bride, notamment dans le cadre de la chirurgie peu invasive, caractérisé en ce que l'instrument (1) présente un plomb (3) muni d'un support de fil (4), ainsi qu'un support de plomb (5) semblable à une tige pouvant être accouplé avec le plomb, et en ce que le support du plomb présente des éléments d'actionnement (7) pour ouvrir ou fermer le support du fil du plomb.

2. Instrument selon la revendication 1, caractérisé en ce que les éléments d'actionnement du support (5) du plomb forment simultanément des éléments d'accouplement pour le relier au plomb (3).

3. Instrument selon la revendication 1 ou 2, caractérisé en ce que le support (4) du fil du plomb (3) présente des éléments de serrage (8) pour immobiliser au moins une extrémité d'une bride ou d'une boucle en fil, et en ce que de préférence une extrémité du fil (12) est fixée au plomb (3) et l'autre extrémité du fil est maintenue par les éléments de serrage (8).

4. Instrument selon la revendication 3, caractérisé en ce que les éléments de serrage (8) sont réalisés ou disposés de façon autobloquante dans le sens inverse au sens de traction du fil (2), et en ce que les éléments de serrage présentent des mâchoires de serrage (9) sollicitées avec ressort dans le sens de serrage, qui sont montées de façon à pivoter l'une par rapport à l'autre et qui peuvent être sollicitées par la tension du fil dans le sens de serrage.

5. Instrument selon la revendication 3 ou 4, caractérisé en ce que les éléments de serrage (8) présentent des saillies d'actionnement (15) pouvant être sollicitées par les éléments d'actionnement du support (5) du plomb pour libérer le fil.

6. Instrument selon l'une des revendications 1 à 5, caractérisé en ce que le plomb (3) présente un canal de passage (11) de préférence central pour permettre le passage d'une extrémité du fil ou d'un instrument de préhension (25).

7. Instrument selon l'une des revendications 1 à 6, caractérisé en ce que le support du plomb présente en tant qu'éléments d'actionnement et de maintien des mâchoires (7) semblables à une pince dont les faces internes sont de préférence adaptées au contour extérieur du plomb (3) et qui, en position fermée, sollicitent les saillies d'actionnement (15) pour libérer le fil.

8. Instrument selon la revendication 7, caractérisé en ce que les mâchoires de prise (7) du support (5) du plomb sont fixées de façon à pivoter à l'extrémité intérieure d'une partie de tige (21), et en ce qu'à l'extrémité extérieure du support du plomb sont prévus des moyens d'actionnement pour ouvrir et fermer les mâchoires de prise (7).

9. Instrument selon l'une des revendications 1 à 8, caractérisé en ce que le support (5) du plomb présente un canal de passage (24) de préférence central qui, lorsque le plomb (3) et le support (5) du plomb sont accouplés, est en alignement avec celui du plomb, et en ce qu'est prévu un davier (25) adapté aux dimensions de ces canaux de passage, qui peut y être introduit par l'extrémité extérieure du support (5) du plomb.

10. Instrument selon la revendication 8 ou 9, caractérisé en ce que le support (5) du plomb présente, en tant que moyen de transmission et d'actionnement pour ouvrir et fermer les mâchoires de prise (7), de préférence un tube interne (22) passant dans la tige de façon coaxiale, à l'extrémité intérieure duquel sont prévus des paliers oscillants (27) pour les mâchoires de prise (7), et en ce que des ailes de traction et de poussée (23) parallèles à l'axe des paliers oscillants et reliées de façon pivotante à la douille extérieure ou à la partie extérieure de la tige (21) sont appliquées notamment sur les mâchoires de prise.

11. Instrument selon l'une des revendications 1 à 10, caractérisé en ce que le plomb (3) est réalisée de façon approximativement cylindrique, avec un canal de passage (11) axial de préférence central, et en ce que les éléments de serrage (8) sont montés de façon pivotante, sont disposés avec leurs mâchoires de serrage (9) dans la zone du canal de passage (11) et dépassent de préférence du pourtour extérieur du plomb (3) avec leurs autres extrémités présentant les saillies d'actionnement (15).

12. Instrument selon l'une des revendications 1 à 11, caractérisé en ce que les éléments de serrage (8) sont sollicités avec ressort en position fermée par au moins une bague en caoutchouc (10) ou analogues logée dans des segments de rainure (18) de saillies (17).

13. Instrument selon l'une des revendications 1 à 12, caractérisé en ce que le plomb (3) présente un canal supplémentaire (19) s'étendant de préférence parallèlement au canal de passage central (11), qui est destiné à l'autre extrémité du fil (12).

14. Instrument notamment selon la revendication 1, caractérisé en ce que les éléments de serrage (8) du support (4) du fil sont réalisés en tant que mâchoires d'écartement et présentent entre eux un canal de passage pour le fil, en ce que sur l'extérieur des mâchoires d'écartement est prévu un cône de serrage coopérant avec une douille de serrage coulissante, en ce que la douille de serrage est sollicitée avec ressort dans le sens de fermeture, et en ce que des points d'application pour le support du plomb sont prévus sur les éléments de serrage, ainsi que sur le cône de serrage, pour ouvrir et fermer les éléments de serrage.

15. Instrument selon la revendication 14, caractérisé en ce que le support du fil présente une douille fendue longitudinalement dans la zone des mâchoires d'écartement et dont la section extérieure s'élargit à cet endroit de préférence de façon conique, sur laquelle est montée de façon coulissante la douille de serrage annulaire.

16. Instrument selon la revendication 14 ou 15, caractérisé en ce que l'une des mâchoires d'écartement, ainsi que la douille de serrage présentent en tant que points d'application pour le support du plomb des prolongements s'étendant approximativement dans la direction axiale, aux extrémités desquels se trouvent des brides d'actionnement espacées en position de serrage et de préférence munies d'orifices de passage.
